# EUROPÄISCHE PATENTSCHRIFT

(11) **EP 4 248 873 B1**
(45) Veröffentlichungstag und Bekanntmachung des Hinweises auf die Patenterteilung: **02.07.2025**
(21) Anmeldenummer: 22192948.2
(22) Anmeldetag: 30.08.2022
(51) Int. Cl.: A61B 6/00, A61B 6/03

(54) **COMPUTERTOMOGRAPHIEGERÄT UND VERFAHREN ZUR ENERGIEÜBERTRAGUNG IN EINEM COMPUTERTOMOGRAPHIEGERÄT**
COMPUTED TOMOGRAPHY APPARATUS AND METHOD FOR TRANSMITTING ENERGY IN A COMPUTED TOMOGRAPHY APPARATUS
APPAREIL DE TOMOGRAPHIE ASSISTÉE PAR ORDINATEUR ET PROCÉDÉ DE TRANSFERT D'ÉNERGIE DANS UN APPAREIL DE TOMOGRAPHIE ASSISTÉE PAR ORDINATEUR

(43) Veröffentlichungstag der Anmeldung: 27.09.2023
(73) Patentinhaber: Siemens Healthineers AG, 91301 Forchheim (DE)
(72) Erfinder: Fehre, Jens, 91353 Hausen (DE); Giebel, Steffen, 91369 Wiesenthau (DE); Köhler, Marco, 91325 Adelsdorf (DE)
(74) Vertreter: Siemens Healthineers Patent Attorneys

(56) Entgegenhaltungen:
- WO-A1-2011/115711
- WO-A1-2014/004447
- US-B2- 8 218 726

## Beschreibung

Bei einer Bildgebungsuntersuchung mittels eines Computertomographiegeräts wird ein Projektionsdatenakquisitionssystem um einen Untersuchungsbereich herum rotierend bewegt, um Projektionsdaten von dem Untersuchungsbereich für verschiedene Projektionsrichtungen zu erfassen. Dazu ist das Projektionsdatenakquisitionssystem in einen rotierenden Gantryteil des Computertomographiegeräts integriert, welcher relativ zu einem tragenden Gantryteil des Computertomographiegeräts drehbar gelagert ist.

Dabei wird zur elektrischen Energieversorgung des Projektionsdatenakquisitionssystems und anderer Komponenten des rotierenden Gantryteils elektrische von dem tragenden Gantryteil zu dem rotierenden Gantryteil übertragen. Eine kontinuierliche Energieversorgung kann beispielsweise mittels einer Schleifringanordnung erfolgen, bei der federnde Schleifkontakte auf ringförmigen Schleifbahnen entlanggeschleift werden und dabei einer Drehbewegung des rotierenden Gantryteils relativ zu dem tragenden Gantryteil folgen.

Eine weitere Möglichkeit, kontinuierlich elektrische Energie von dem tragenden Gantryteil zu dem rotierenden Gantryteil zu übertragen, basiert auf einer kontaktlosen Drehverbindung, beispielsweise basierend auf induktiver, kapazitiver und/oder elektromagnetischer Kopplung mit wenigstens einer ringförmigen Leiteranordnung.

Ringförmige Schleifbahnen und/oder Koppelleiter können die Flexibilität, mit der Bauraum im Computertomographiegerät, insbesondere auf dem Rotor, für andere Komponenten verfügbar ist, einschränken. Die Abnutzung von Schleifkontakten bedeutet einen erhöhten Wartungsaufwand. Für kontaktlose Drehverbindungen ist der Fertigungsaufwand relativ hoch, insbesondere in Bezug auf das Spaltmaß zwischen dem rotierenden Gantryteil und dem tragenden Gantryteil im Bereich der kontaktlosen Drehverbindung.

US 8 218 726 B2 und US 10 342 506 B2 offenbaren jeweils ein Computertomographiegerät mit einem Energiespeicher. Als Stand der Technik sind hierbei ferner die WO 2014/004447 A1 und WO 2011/115711 A1 zu nennen.

Die Erfindung hat die Aufgabe eine Alternative zu herkömmlichen Energieübertragungslösungen für Computertomographiegeräte bereitzustellen.

Die Erfindung betrifft ein Computertomographiegerät, aufweisend eine Gantry mit einem tragenden Gantryteil und einem Rotor, wobei der Rotor ein Projektionsdatenakquisitionssystem und einen Energiespeicher zur Energieversorgung des Projektionsdatenakquisitionssystems aufweist und relativ zu dem tragenden Gantryteil drehbar gelagert ist, wobei die Gantry ein Energieübertragungssystem aufweist, welches für eine Energieübertragung von dem tragenden Gantryteil zu dem Energiespeicher des Rotors eingerichtet ist, wobei während der Energieübertragung von dem tragenden Gantryteil zu dem Energiespeicher des Rotors der Rotor relativ zu dem tragenden Gantryteil ruht.

Die Gantry des Computertomographiegeräts kann insbesondere einen Anschluss zum Empfangen von elektrischer Energie aufweisen. Die elektrische Energie kann beispielsweise von einem elektrischen Energieübertragungsnetz, insbesondere von einem Niederspannungsnetz einer Klinik, bereitgestellt werden. Der Anschluss zum Empfangen der elektrischen Energie kann insbesondere zum lösbaren Verbinden mit dem elektrischen Energieübertragungsnetz, beispielsweise mittels eines Anschlusskabels, eingerichtet sein.

Insbesondere kann die Energieübertragung von dem tragenden Gantryteil zu dem Energiespeicher des Rotors derart erfolgen, dass dabei ein Speichern von elektrischer Energie in dem Energiespeicher des Rotors erfolgt. Der Energiespeicher des Rotors kann insbesondere zur Energieversorgung des Projektionsdatenakquisitionssystems derart eingerichtet sein, dass das Projektionsdatenakquisitionssystem mit elektrischer Energie aus dem Energiespeicher des Rotors versorgt werden kann, während sich der Rotor relativ zu dem tragenden Gantryteil dreht.

Insbesondere kann vorgesehen sein, dass durch die Energieübertragung von dem tragenden Gantryteil zu dem Energiespeicher des Rotors eine elektrische Energie, die zum Erfassen von Projektionsdaten mittels des Projektionsdatenakquisitionssystems für wenigstens ein computertomographisches Schnittbild erforderlich ist, von dem tragenden Gantryteil zu dem Energiespeicher des Rotors übertragen wird.

Der Energiespeicher des Rotors kann beispielsweise auf einem Akkumulator und/oder auf Kondensatoren, insbesondere Superkondensatoren, basieren. Superkondensatoren sind auch unter den Begriffen elektrochemischer Kondensator, Ultrakondensator, Ultracap und Supercap bekannt.

Das Projektionsdatenakquisitionssystem kann beispielsweise eine Röntgenquelle und/oder einen Röntgendetektor, welcher mit der Röntgenquelle zusammenwirkt, aufweisen und/oder zum Erfassen von Projektionsdaten eingerichtet sein. Das Erfassen der Projektionsdaten mittels des Projektionsdatenakquisitionssystems kann dabei insbesondere ein Erzeugen von Röntgenstrahlen mittels der Röntgenquelle und ein Detektieren der Röntgenstrahlen mittels des Röntgendetektors umfassen. Hiermit wird ferner ein medizinisches Bildgebungsgerät offenbart, aufweisend eine Gantry mit einem tragenden Gantryteil und einem bewegbaren Gantryteil, wobei das bewegbare Gantryteil ein Projektionsdatenakquisitionssystem und einen Energiespeicher zur Energieversorgung des Projektionsdatenakquisitionssystems aufweist und relativ zu dem tragenden Gantryteil bewegbar gelagert ist, wobei die Gantry ein Energieübertragungssystem aufweist, welches für eine Energieübertragung von dem tragenden Gantryteil zu dem Energiespeicher des bewegbaren Gantryteils eingerichtet ist, wobei während der Energieübertragung von dem tragenden Gantryteil zu dem Energiespeicher des bewegbaren Gantryteils das bewegbare Gantryteil relativ zu dem tragenden Gantryteil ruht. Weiterhin kann vorgesehen sein, dass das Energieübertragungssystem ferner für eine Energieübertragung von dem Energiespeicher des bewegbaren Gantryteils zu dem tragenden Gantryteil eingerichtet ist, wobei während der Energieübertragung von dem Energiespeicher des bewegbaren Gantryteils zu dem tragenden Gantryteil das bewegbare Gantryteil relativ zu dem tragenden Gantryteil ruht.

Beispielweise kann das medizinische Bildgebungsgerät ein C-Bogen-Röntgengerät sein, wobei der bewegbare Gantryteil ein C-Bogen des C-Bogen-Röntgengeräts ist. Insbesondere kann das medizinische Bildgebungsgerät mit geeigneten Merkmalen, die in Bezug auf den Rotor der Gantry des Computertomographiegeräts beschrieben sind, weitergebildet werden, indem der Bezug auf den Rotor der Gantry des Computertomographiegeräts durch einen Bezug auf den bewegbaren Gantryteil des medizinischen Bildgebungsgeräts ersetzt wird.

Das Computertomographiegerät kann insbesondere ein mobiles Computertomographiegerät oder ein stationäres Computertomographiegerät sein. Das Computertomographiegerät kann insbesondere als Kopf-Computertomographiegerät für eine Kopfbildgebung ausgebildet sein, beispielsweise als mobiles Kopf-Computertomographiegerät ausgebildet sein.

Die Gantry kann insbesondere eine Öffnung aufweisen. Die Öffnung kann beispielsweise derart ausgebildet sein, dass für eine Untersuchung mittels des Computertomographiegeräts ein Untersuchungsobjekt entlang einer Systemachse der Gantry in die Öffnung eingeführt werden kann. Das Untersuchungsobjekt kann beispielsweise ein Mensch, ein Tier oder ein Phantom sein. Der Rotor kann insbesondere relativ zu dem tragenden Gantryteil um eine Drehachse drehbar gelagert sein. Die Drehachse kann beispielsweise gleich der Systemachse sein.

Das Computertomographiegerät kann somit derart betrieben werden, dass eine Energieübertragung zwischen dem Rotor und dem tragenden Gantryteil nur in einem Ruhezustand des Rotors relativ zu dem tragenden Gantryteil, insbesondere zwischen zwei Betriebszuständen des Computertomographiegeräts, in denen sich der Rotor relativ zu dem tragenden Gantryteil dreht, erfolgt. Eine kontinuierliche Energieübertragung während einer Drehung des Rotors relativ zu dem tragenden Gantryteil ist somit nicht erforderlich. Daher kann auf ringförmige Schleifbahnen und Koppelleiter verzichtet werden. Im Vergleich zu herkömmlichen ringbasierten Energieübertragungslösungen für Computertomographiegeräte kann der Aufwand für Fertigung und Wartung somit verringert werden und der Bauraum im Computertomographiegerät flexibler genutzt werden. Weiterhin können auf diese Weise Materialkosten und Gewicht des Computertomographiegeräts reduziert werden.

Erfindungsgemäß ist vorgesehen, dass das Energieübertragungssystem ferner für eine Energieübertragung von dem Energiespeicher des Rotors zu dem tragenden Gantryteil eingerichtet ist, wobei während der Energieübertragung von dem Energiespeicher des Rotors zu dem tragenden Gantryteil der Rotor relativ zu dem tragenden Gantryteil ruht. Beispielsweise kann der Energiespeicher des Rotors zur Energieversorgung des gesamten Computertomographiegeräts genutzt werden, während der Rotor relativ zu dem tragenden Gantryteil ruht.

Eine Ausführungsform sieht vor, dass die Gantry ein Fahrwerk aufweist, welches für eine Fahrbewegung der Gantry relativ zu einer Grundfläche eingerichtet ist, wobei die Energieübertragung von dem Energiespeicher des Rotors zu dem tragenden Gantryteil während der Fahrbewegung der Gantry erfolgt.

Die Grundfläche kann beispielsweise ein Boden eines Untersuchungsraumes sein und/oder von einem Bodenbelag und/oder einer Unterlage gebildet sein. Das Fahrwerk kann insbesondere dazu eingerichtet sein, die Gantry parallel zu der Grundfläche relativ zu der Grundfläche zu bewegen.

Das Fahrwerk kann beispielsweise auf Rädern basieren und/oder omnidirektional sein. Die Fahrbewegung der Gantry kann insbesondere erfolgen, während der Rotor relativ zu dem tragenden Gantryteil ruht. Die Fahrbewegung der Gantry kann beispielsweise erfolgen, um die Gantry innerhalb eines Untersuchungsraumes und/oder zwischen verschiedenen Untersuchungsräumen einer Klinik zu bewegen. Die Fahrbewegung der Gantry kann insbesondere erfolgen, während die Gantry von einem elektrischen Energieübertragungsnetz, insbesondere von einem Niederspannungsnetz einer Klinik, getrennt ist.

Eine Ausführungsform sieht vor, dass der tragende Gantryteil einen elektrischen Fahrantrieb, der mit dem Fahrwerk zusammenwirkt, aufweist, wobei die Energieübertragung von dem Energiespeicher des Rotors zu dem tragenden Gantryteil mittels des Energieübertragungssystems von dem Energiespeicher des Rotors zu dem elektrischen Fahrantrieb erfolgt, um die Fahrbewegung der Gantry mittels des Fahrantriebs anzutreiben.

Wenn die elektrische Energie für die Fahrbewegung der Gantry dem Energiespeicher des Rotors entnommen wird, braucht auf dem tragenden Gantryteil keine elektrische Energie für die Fahrbewegung der Gantry gespeichert zu werden. Somit können Bauraum und Kosten für einen zur Energieversorgung des Fahrwerks ausgelegten Energiespeicher auf dem tragenden Gantryteil eingespart werden. Redundanzen in den Energiespeicherkapazitäten können eingespart werden, wenn sowohl die Energieversorgung des Fahrwerks als auch die Energieversorgung des Projektionsdatenakquisitionssystems mittels des Energiespeichers des Rotors erfolgen.

Eine Ausführungsform sieht vor, dass der tragende Gantryteil einen Energiespeicher aufweist, wobei die Energieübertragung von dem tragenden Gantryteil zu dem Energiespeicher des Rotors mittels des Energieübertragungssystems von dem Energiespeicher des tragenden Gantryteils zu dem Energiespeicher des Rotors erfolgt und/oder wobei die Energieübertragung von dem Energiespeicher des Rotors zu dem tragenden Gantryteil mittels des Energieübertragungssystems von dem Energiespeicher des Rotors zu dem Energiespeicher des tragenden Gantryteils erfolgt.

Insbesondere kann vorgesehen sein, dass die Energieübertragung von dem Energiespeicher des tragenden Gantryteils zu dem Energiespeicher des Rotors während der Fahrbewegung der Gantry erfolgt. Der Energiespeicher des tragenden Gantryteils kann für eine Energieversorgung von Komponenten des tragenden Gantryteils verwendet werden, während sich der Rotor relativ zu dem tragenden Gantryteil dreht und/oder während der Energiespeicher des Rotors nicht mittels des Energieübertragungssystems mit dem tragenden Gantryteil verbunden ist.

Das Fahrwerk kann beispielsweise für eine Scanbewegung der Gantry relativ zu dem Untersuchungsobjekt während des Erfassens der Projektionsdaten eingerichtet sein. Insbesondere kann dabei das Untersuchungsobjekt relativ zu der Grundfläche ruhen. Die Scanbewegung der Gantry kann dabei insbesondere entlang der Systemachse der Gantry erfolgen. Die Scanbewegung kann beispielsweise basierend auf elektrischer Energie aus dem Energiespeicher des tragenden Gantryteils angetrieben werden.

Die Gantry, insbesondere das Energieübertragungssystem der Gantry, kann beispielsweise eine Schaltvorrichtung aufweisen. Die Schaltvorrichtung kann beispielsweise zum Verteilen von elektrischer Energie auf den Energiespeicher des tragenden Gantryteils und den Energiespeicher des Rotors eingerichtet sein. Die Schaltvorrichtung kann beispielsweise zum Schalten des Computertomographiegeräts in verschiedene Betriebszustände, die sich voneinander in Bezug auf den Fluss der elektrischen Energie unterscheiden, eingerichtet sein.

Weiterhin kann vorgesehen sein, dass der tragende Gantryteil ein Datenverarbeitungssystem aufweist, wobei der Energiespeicher des tragenden Gantryteils für eine Energieversorgung des Datenverarbeitungssystems eingerichtet ist, wobei die Energieversorgung des Datenverarbeitungssystems mit elektrischer Energie aus dem Energiespeicher des tragenden Gantryteils erfolgt, insbesondere während sich der Rotor relativ zu dem tragenden Gantryteil dreht.

Das Datenverarbeitungssystem kann beispielsweise zur Steuerung des Computertomographiegeräts und/oder zur Bildverarbeitung der Projektionsdaten eingerichtet sein.

Eine Ausführungsform sieht vor, dass der tragende Gantryteil ein erstes Verbindungselement des Energieübertragungssystems aufweist, dass der Rotor ein zweites Verbindungselement des Energieübertragungssystems aufweist und dass das erste Verbindungselement und das zweite Verbindungselement zueinander für ein Herstellen einer Energieübertragungsverbindung korrespondierend ausgebildet sind. Insbesondere kann vorgesehen sein, dass das erste Verbindungselement und das zweite Verbindungselement zueinander für das Herstellen der Energieübertragungsverbindung korrespondierend ausgerichtet sind, wenn sich der Rotor in einer Kopplungs-Winkelposition relativ zu dem tragenden Gantryteil befindet.

Insbesondere kann vorgesehen sein, dass die Gantry eine Winkelpositionierungsvorrichtung aufweist, wobei die Winkelpositionierungsvorrichtung zur Winkelpositionierung des Rotors in der Kopplungs-Winkelposition relativ zu dem tragenden Gantryteil eingerichtet ist.

Insbesondere kann vorgesehen sein, dass die Energieübertragung von dem tragenden Gantryteil zu dem Energiespeicher des Rotors mittels der Energieübertragungsverbindung erfolgt und/oder dass die Energieübertragung von dem Energiespeicher des Rotors zu dem tragenden Gantryteil mittels der Energieübertragungsverbindung erfolgt. Die Energieübertragungsverbindung kann insbesondere bidirektional sein.

Insbesondere kann vorgesehen sein, dass während der Energieübertragung von dem tragenden Gantryteil zu dem Energiespeicher des Rotors der Rotor relativ zu dem tragenden Gantryteil ruht und sich in der Kopplungs-Winkelposition relativ zu dem tragenden Gantryteil befindet und/oder dass während der Energieübertragung von dem Energiespeicher des Rotors zu dem tragenden Gantryteil der Rotor relativ zu dem tragenden Gantryteil ruht und sich in der Kopplungs-Winkelposition relativ zu dem tragenden Gantryteil befindet.

Die Kopplungs-Winkelposition kann beispielsweise auf die Drehachse, um welche der Rotor relativ zu dem tragenden Gantryteil drehbar gelagert ist, bezogen sein. Die Kopplungs-Winkelposition kann beispielsweise diejenige Winkelposition des Rotors relativ zu dem tragenden Gantryteil sein, in welcher ein Abstand zwischen dem ersten Verbindungselement und dem zweiten Verbindungselement im Vergleich zu anderen Winkelpositionen des Rotors relativ zu dem tragenden Gantryteil, die bei einer Drehung des Rotors relativ zu dem tragenden Gantryteil durchlaufen werden, minimal ist.

Insbesondere kann vorgesehen sein, dass die Gantry eine Winkelpositionierungsvorrichtung aufweist, wobei die Winkelpositionierungsvorrichtung zur Winkelpositionierung des Rotors in der Kopplungs-Winkelposition relativ zu dem tragenden Gantryteil eingerichtet ist.

Die Winkelpositionierung des Rotors kann beispielsweise basierend auf einem Abbremsen einer Drehbewegung des Rotors relativ zu dem tragenden Gantryteil mittels einer Bremse derart erfolgen, dass die Drehbewegung des Rotors in der Kopplungs-Winkelposition relativ zu dem tragenden Gantryteil endet. Die Winkelpositionierungsvorrichtung kann beispielsweise die Bremse aufweisen. Die Winkelpositionierungsvorrichtung kann beispielsweise zum Antreiben einer Winkelpositionierungsdrehbewegung des Rotors relativ zu dem tragenden Gantryteil eingerichtet sein. Das Abbremsen der Drehbewegung des Rotors, insbesondere der Winkelpositionierungsdrehbewegung des Rotors, und/oder das Antreiben der Drehbewegung des Rotors, insbesondere der Winkelpositionierungsdrehbewegung des Rotors, kann beispielsweise mit elektrischer Energie aus dem Energiespeicher des Rotors und/oder mit elektrischer Energie aus dem Energiespeicher des tragenden Gantryteils erfolgen.

Die Winkelpositionierungsvorrichtung kann beispielsweise eine Fixiervorrichtung aufweisen, die zum Fixieren des Rotors relativ zu dem tragenden Gantryteil in der Kopplungs-Winkelposition relativ zu dem tragenden Gantryteil eingerichtet ist.

Die Winkelpositionierungsvorrichtung kann beispielsweise einen gefederten Bolzen auf dem tragenden Gantryteil und ein Loch auf dem Rotor aufweisen, welche derart ausgebildet und angeordnet sind, dass der Bolzen in das Loch einrasten kann, wenn sich der Rotor in der Kopplungs-Winkelposition relativ zu dem tragenden Gantryteil befindet, und damit den Rotor in der Kopplungs-Winkelposition relativ zu dem tragenden Gantryteil fixieren kann.

Eine Ausführungsform sieht vor, dass die Energieübertragungsverbindung eine elektrische Steckverbindung des ersten Verbindungselements und des zweiten Verbindungselements miteinander ist.

Eine Ausführungsform sieht vor, dass die Energieübertragungsverbindung zur kontaktlosen elektrischen Energieübertragung zwischen dem ersten Verbindungselement und dem zweiten Verbindungselement eingerichtet ist, beispielsweise zur kontaktlosen induktiven elektrischen Energieübertragung zwischen dem ersten Verbindungselement und dem zweiten Verbindungselement und/oder zur kontaktlosen kapazitiven elektrischen Energieübertragung zwischen dem ersten Verbindungselement und dem zweiten Verbindungselement eingerichtet ist.

Insbesondere kann vorgesehen sein, dass die Energieübertragung von dem tragenden Gantryteil zu dem Energiespeicher des Rotors die kontaktlose elektrische Energieübertragung zwischen dem ersten Verbindungselement und dem zweiten Verbindungselement aufweist und/oder dass die Energieübertragung von dem Energiespeicher des Rotors zu dem tragenden Gantryteil die kontaktlose elektrische Energieübertragung zwischen dem ersten Verbindungselement und dem zweiten Verbindungselement aufweist.

Die kontaktlose elektrische Energieübertragung zwischen dem ersten Verbindungselement und dem zweiten Verbindungselement kann auf verschleißarme Weise realisiert werden und/oder zur galvanischen Trennung des Rotors von dem tragenden Gantryteil verwendet werden.

Weiterhin kann vorgesehen sein, dass die kontaktlose elektrische Energieübertragung zwischen dem ersten Verbindungselement und dem zweiten Verbindungselement auch erfolgen kann, während sich der Rotor relativ zu dem tragenden Gantryteil dreht.

Eine Ausführungsform sieht vor, dass die Gantry eine Kopplungsvorrichtung aufweist, welche zum Anordnen des ersten Verbindungselements und des zweiten Verbindungselements in einer Kopplungs-Position relativ zueinander, während der Rotor relativ zu dem tragenden Gantryteil ruht, insbesondere während der Rotor in der Kopplungs-Winkelposition relativ zu dem tragenden Gantryteil ruht, und zum Anordnen des ersten Verbindungselements und des zweiten Verbindungselements in einer Entkopplungs-Position relativ zueinander, während der Rotor relativ zu dem tragenden Gantryteil ruht, insbesondere während der Rotor in der Kopplungs-Winkelposition relativ zu dem tragenden Gantryteil ruht, eingerichtet ist.

Insbesondere kann vorgesehen sein, dass die Energieübertragungsverbindung durch eine Kopplung des ersten Verbindungselements und des zweiten Verbindungselements aneinander hergestellt ist, wenn sich der Rotor in der Kopplungs-Winkelposition relativ zu dem tragenden Gantryteil befindet und wenn das erste Verbindungselement und das zweite Verbindungselement in der Kopplungs-Position relativ zueinander angeordnet sind. Die Kopplung des ersten Verbindungselements und des zweiten Verbindungselements aneinander kann beispielsweise induktiv und/oder kapazitiv sein oder auf einem elektrischen Kontakt, beispielsweise einem gefederten und/oder gesteckten elektrischen Kontakt, des ersten Verbindungselements und des zweiten Verbindungselements miteinander basieren.

Insbesondere kann vorgesehen sein, dass die Energieübertragungsverbindung durch eine Entkopplung des ersten Verbindungselements und des zweiten Verbindungselements voneinander unterbrochen ist, wenn das erste Verbindungselement und das zweite Verbindungselement in der Entkopplungs-Position relativ zueinander angeordnet sind.

Die Kopplung kann beispielsweise elektrisch und/oder mechanisch sein. Die Entkopplung kann beispielsweise elektrisch und/oder mechanisch sein. Die Entkopplung des ersten Verbindungselements und des zweiten Verbindungselements voneinander kann beispielsweise erfolgen, indem ein Spalt zwischen dem ersten Verbindungselement und dem zweiten Verbindungselement geöffnet und/oder vergrößert wird. Durch das Öffnen und/oder vergrößern des Spalts kann beispielsweise eine Drehung des Rotors relativ zu dem tragenden Gantryteil ermöglicht werden, ohne dass die Drehung des Rotors durch die Kopplung des ersten Verbindungselements und des zweiten Verbindungselements aneinander verhindert und/oder gestört wird.

Wenn die Energieübertragungsverbindung eine elektrische Steckverbindung des ersten Verbindungselements und des zweiten Verbindungselements ist, kann die Entkopplung des ersten Verbindungselements und des zweiten Verbindungselements voneinander durch ein Lösen der Steckverbindung erfolgen.

Das Anordnen des ersten Verbindungselements und des zweiten Verbindungselements in der Kopplungs-Position relativ zueinander und/oder das Anordnen des ersten Verbindungselements und des zweiten Verbindungselements in der Entkopplungs-Position relativ zueinander kann beispielsweise mit elektrischer Energie aus dem Energiespeicher des Rotors und/oder mit elektrischer Energie aus dem Energiespeicher des tragenden Gantryteils erfolgen.

Die Erfindung betrifft ferner ein Verfahren zur Energieübertragung in einem Computertomographiegerät, wobei das Computertomographiegerät eine Gantry mit einem tragenden Gantryteil, einem Rotor und einem Energieübertragungssystem aufweist, wobei der Rotor das Projektionsdatenakquisitionssystem und einen Energiespeicher aufweist und relativ zu dem tragenden Gantryteil drehbar gelagert ist, das Verfahren umfassend:
- ein Herbeiführen eines Ruhezustands des Rotors relativ zu dem tragenden Gantryteil derart, dass der Rotor relativ zu dem tragenden Gantryteil ruht,
- ein Durchführen einer Energieübertragung von dem tragenden Gantryteil zu dem Energiespeicher des Rotors mittels des Energieübertragungssystems, während der Rotor relativ zu dem tragenden Gantryteil ruht,
- ein Drehen des Rotors relativ zu dem tragenden Gantryteil,
- ein Versorgen des Projektionsdatenakquisitionssystems mit elektrischer Energie aus dem Energiespeicher des Rotors während des Drehens des Rotors relativ zu dem tragenden Gantryteil,
- dadurch gekennzeichnet, dass eine Energieübertragung von dem Energiespeicher des Rotors zu dem tragenden Gantryteil mittels des Energieübertragungssystems durchgeführt wird, während der Rotor relativ zu dem tragenden Gantryteil ruht.

Insbesondere kann die Energieübertragung von dem tragenden Gantryteil zu dem Energiespeicher des Rotors derart durchgeführt werden, dass dabei elektrische Energie in dem Energiespeicher des Rotors gespeichert wird.

Eine Ausführungsform sieht vor, dass eine Fahrbewegung der Gantry relativ zu einer Grundfläche mittels eines Fahrwerks durchgeführt wird, wobei die Energieübertragung von dem Energiespeicher des Rotors zu dem tragenden Gantryteil während der Fahrbewegung der Gantry durchgeführt wird.

Eine Ausführungsform sieht vor, dass durch die Energieübertragung von dem Energiespeicher des Rotors zu dem tragenden Gantryteil mittels des Energieübertragungssystems elektrische Energie für einen elektrischen Fahrantrieb, der mit dem Fahrwerk zusammenwirkt, von dem Energiespeicher des Rotors zu dem tragenden Gantryteil übertragen wird. Insbesondere kann die Fahrbewegung der Gantry mittels des elektrischen Fahrantriebs basierend auf der elektrischen Energie für den elektrischen Fahrantrieb angetrieben werden.

Eine Ausführungsform sieht vor, dass der tragende Gantryteil einen Energiespeicher aufweist, wobei die Energieübertragung von dem tragenden Gantryteil zu dem Energiespeicher des Rotors mittels des Energieübertragungssystems von dem Energiespeicher des tragenden Gantryteils zu dem Energiespeicher des Rotors durchgeführt wird und/oder wobei die Energieübertragung von dem Energiespeicher des Rotors zu dem tragenden Gantryteil mittels des Energieübertragungssystems von dem Energiespeicher des Rotors zu dem Energiespeicher des tragenden Gantryteils durchgeführt wird.

Eine Ausführungsform sieht vor, dass der tragende Gantryteil ein erstes Verbindungselement des Energieübertragungssystems aufweist, wobei der Rotor ein zweites Verbindungselement des Energieübertragungssystems aufweist, wobei das erste Verbindungselement und das zweite Verbindungselement zueinander für ein Herstellen einer Energieübertragungsverbindung korrespondierend ausgebildet sind. Insbesondere kann der Rotor in einer Kopplungs-Winkelposition relativ zu dem tragenden Gantryteil derart winkelpositioniert werden, insbesondere mittels einer Winkelpositionierungsvorrichtung derart winkelpositioniert werden, dass das erste Verbindungselement und das zweite Verbindungselement zueinander für das Herstellen der Energieübertragungsverbindung korrespondierend ausgerichtet sind.

Eine Ausführungsform sieht vor, dass die Energieübertragungsverbindung durch eine Kopplung des ersten Verbindungselements und des zweiten Verbindungselements aneinander hergestellt wird, beispielsweise indem das erste Verbindungselement und das zweite Verbindungselement in einer Kopplungs-Position relativ zueinander angeordnet werden, insbesondere mittels einer Kopplungsvorrichtung in der Kopplungs-Position relativ zueinander angeordnet werden, während der Rotor relativ zu dem tragenden Gantryteil ruht und sich in der Kopplungs-Winkelposition relativ zu dem tragenden Gantryteil befindet.

Insbesondere kann vorgesehen sein, dass die Energieübertragungsverbindung durch eine Entkopplung des ersten Verbindungselements und des zweiten Verbindungselements voneinander unterbrochen wird, beispielsweise indem das erste Verbindungselement und das zweite Verbindungselement in einer Entkopplungs-Position relativ zueinander angeordnet werden, insbesondere mittels der Kopplungsvorrichtung in der Entkopplungs-Position relativ zueinander angeordnet werden, während der Rotor relativ zu dem tragenden Gantryteil ruht und sich in der Kopplungs-Winkelposition relativ zu dem tragenden Gantryteil befindet.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
Die Fig. 1 zeigt ein Energieflussdiagramm für einen ersten Betriebszustand des Computertomographiegeräts.
Die Fig. 2 zeigt ein Energieflussdiagramm für einen zweiten Betriebszustand des Computertomographiegeräts.
Die Fig. 3 zeigt ein Energieflussdiagramm für einen dritten Betriebszustand des Computertomographiegeräts.
Die Fig. 4 zeigt ein Energieflussdiagramm für einen vierten Betriebszustand des Computertomographiegeräts.
Die Fig. 5 zeigt ein Energieflussdiagramm für einen fünften Betriebszustand des Computertomographiegeräts.
Die Fig. 6 zeigt ein Energieflussdiagramm für einen Betriebszustand eines Computertomographiegeräts gemäß einem weiteren Beispiel.
Die Fig. 7 zeigt ein Energieflussdiagramm für einen Betriebszustand eines Computertomographiegeräts gemäß einem weiteren Beispiel.
Die Fig. 8 zeigt ein Beispiel für ein Computertomographiegerät.
Die Fig. 9 zeigt ein Ablaufdiagramm eines Verfahrens zur Energieübertragung in einem Computertomographiegerät.

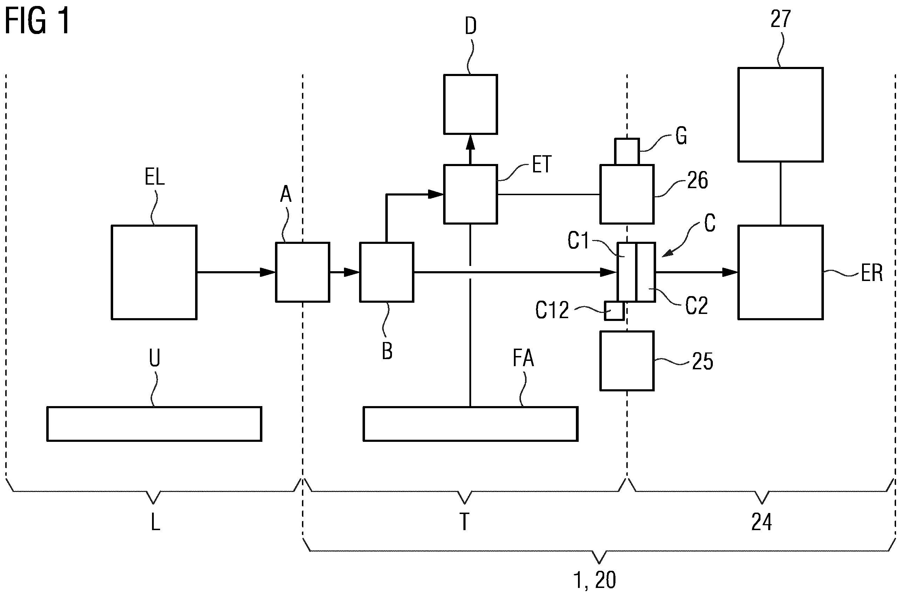

Die Fig. 1 zeigt ein Energieflussdiagramm für einen ersten Betriebszustand des Computertomographiegeräts 1. Das Computertomographiegerät 1 weist eine Gantry 20 mit einem tragenden Gantryteil T und einem Rotor 24 auf, wobei der Rotor 24 ein Projektionsdatenakquisitionssystem 27 und einen Energiespeicher ER zur Energieversorgung des Projektionsdatenakquisitionssystems 27 aufweist und relativ zu dem tragenden Gantryteil T drehbar gelagert ist, wobei die Gantry 20 ein Energieübertragungssystem C aufweist, welches für eine Energieübertragung von dem tragenden Gantryteil T zu dem Energiespeicher ER des Rotors 24 eingerichtet ist, wobei während der Energieübertragung von dem tragenden Gantryteil T zu dem Energiespeicher ER des Rotors 24 der Rotor 24 relativ zu dem tragenden Gantryteil T ruht.

In dem ersten Betriebszustand des Computertomographiegeräts 1 ruht der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C hergestellt ist.

Die Gantry 20 des Computertomographiegeräts 1 weist einen Anschluss A zum Empfangen von elektrischer Energie auf. Die elektrische Energie wird von einem elektrischen Energieübertragungsnetz EL, beispielsweise einem Niederspannungsnetz einer Klinik, bereitgestellt. In dem ersten Betriebszustand des Computertomographiegeräts 1 ist der Anschluss A mittels eines Anschlusskabels lösbar mit dem elektrischen Energieübertragungsnetz EL verbunden.

Der tragende Gantryteil T weist einen Energiespeicher ET auf, wobei die Energieübertragung von dem tragenden Gantryteil T zu dem Energiespeicher ER des Rotors 24 mittels des Energieübertragungssystems C von dem Energiespeicher ET des tragenden Gantryteils T zu dem Energiespeicher ER des Rotors 24 erfolgt und/oder wobei die Energieübertragung von dem Energiespeicher ER des Rotors 24 zu dem tragenden Gantryteil T mittels des Energieübertragungssystems C von dem Energiespeicher ER des Rotors 24 zu dem Energiespeicher ET des tragenden Gantryteils T erfolgt.

Der tragende Gantryteil T weist ein Datenverarbeitungssystem D auf, wobei der Energiespeicher ET des tragenden Gantryteils T für eine Energieversorgung des Datenverarbeitungssystems D eingerichtet ist, wobei die Energieversorgung des Datenverarbeitungssystems D mit elektrischer Energie aus dem Energiespeicher ET des tragenden Gantryteils T erfolgt, insbesondere während sich der Rotor 24 relativ zu dem tragenden Gantryteil T dreht.

Das Energieübertragungssystem C weist die Schaltvorrichtung B auf. Die von dem elektrischen Energieübertragungsnetz EL empfangene elektrische Energie wird mittels der Schaltvorrichtung B auf den Energiespeicher ET des tragenden Gantryteils T und den Energiespeicher ER des Rotors 24 verteilt. In dem ersten Betriebszustand des Computertomographiegeräts 1 wird somit sowohl in dem Energiespeicher ET des tragenden Gantryteils T als auch in dem Energiespeicher ER des Rotors 24 jeweils elektrische Energie gespeichert.

Der tragende Gantryteil T weist ein erstes Verbindungselement C1 des Energieübertragungssystems C auf. Der Rotor 24 weist ein zweites Verbindungselement C2 des Energieübertragungssystems C auf. Das erste Verbindungselement C1 und das zweite Verbindungselement C2 sind zueinander für ein Herstellen einer Energieübertragungsverbindung korrespondierend ausgebildet. Das erste Verbindungselement C1 und das zweite Verbindungselement C2 sind zueinander für das Herstellen der Energieübertragungsverbindung korrespondierend ausgerichtet, wenn sich der Rotor 24 in einer Kopplungs-Winkelposition relativ zu dem tragenden Gantryteil T befindet.

Die Gantry 20 weist die Winkelpositionierungsvorrichtung G auf, wobei die Winkelpositionierungsvorrichtung G zur Winkelpositionierung des Rotors 24 in der Kopplungs-Winkelposition relativ zu dem tragenden Gantryteil T eingerichtet ist.

Die Energieübertragungsverbindung kann beispielsweise eine elektrische Steckverbindung des ersten Verbindungselements C1 und des zweiten Verbindungselements C2 miteinander sein. Alternativ dazu kann die Energieübertragungsverbindung beispielsweise zur kontaktlosen elektrischen Energieübertragung zwischen dem ersten Verbindungselement C1 und dem zweiten Verbindungselement C2 eingerichtet sein.

Die Gantry 20 weist eine Kopplungsvorrichtung C12 auf, welche zum Anordnen des ersten Verbindungselements C1 und des zweiten Verbindungselements C2 in einer Kopplungs-Position relativ zueinander, während der Rotor 24 relativ zu dem tragenden Gantryteil T ruht, und zum Anordnen des ersten Verbindungselements C1 und des zweiten Verbindungselements C2 in einer Entkopplungs-Position relativ zueinander, während der Rotor 24 relativ zu dem tragenden Gantryteil T ruht, eingerichtet ist.

Die Energieübertragungsverbindung ist durch eine Kopplung des ersten Verbindungselements C1 und des zweiten Verbindungselements C2 aneinander hergestellt, wenn sich der Rotor 24 in der Kopplungs-Winkelposition relativ zu dem tragenden Gantryteil T befindet und wenn das erste Verbindungselement C1 und das zweite Verbindungselement C2 in der Kopplungs-Position relativ zueinander angeordnet sind.

Die Energieübertragungsverbindung ist durch eine Entkopplung des ersten Verbindungselements C1 und des zweiten Verbindungselements C2 voneinander unterbrochen, wenn das erste Verbindungselement C1 und das zweite Verbindungselement C2 in der Entkopplungs-Position relativ zueinander angeordnet sind.

In dem ersten Betriebszustand des Computertomographiegeräts 1 ist die Energieübertragungsverbindung durch eine Kopplung des ersten Verbindungselements C1 und des zweiten Verbindungselements C2 aneinander hergestellt, indem das erste Verbindungselement C1 und das zweite Verbindungselement C2 mittels der Kopplungsvorrichtung C12 in einer Kopplungs-Position relativ zueinander angeordnet sind. Der Rotor 24 ruht relativ zu dem tragenden Gantryteil T und befindet sich in der Kopplungs-Winkelposition relativ zu dem tragenden Gantryteil T.

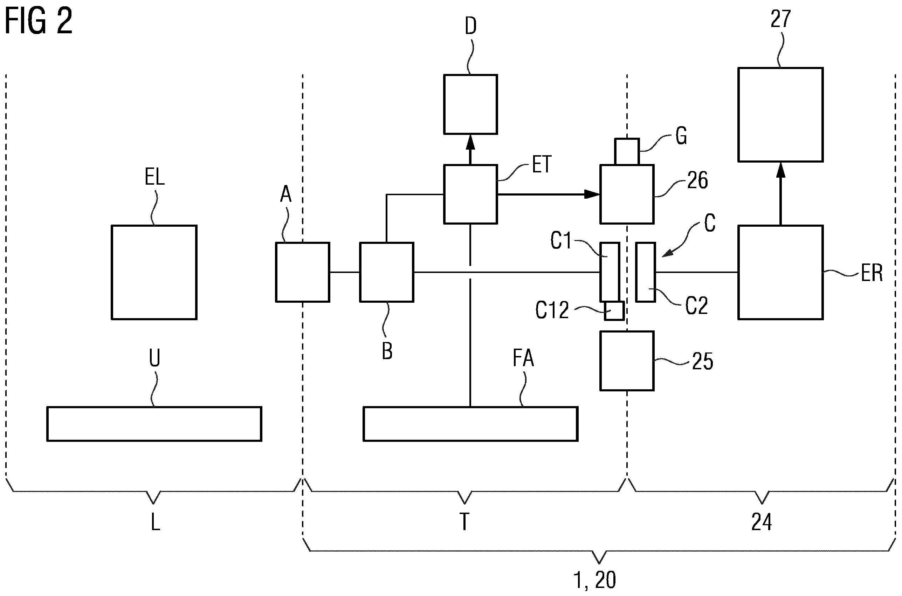

Die Fig. 2 zeigt ein Energieflussdiagramm für einen zweiten Betriebszustand des Computertomographiegeräts 1. In dem zweiten Betriebszustand des Computertomographiegeräts 1 dreht sich der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C unterbrochen ist. In dem zweiten Betriebszustand des Computertomographiegeräts 1 ist der Anschluss A nicht mit dem elektrischen Energieübertragungsnetz EL verbunden.

Es werden Projektionsdaten mittels des Projektionsdatenakquisitionssystems 27 erfasst, wobei das Projektionsdatenakquisitionssystem 27 mit elektrischer Energie von dem Energiespeicher ER des Rotors 24 versorgt wird. Die Gantry 20 weist den Drehantrieb 26 zum Antreiben der Drehbewegung des Rotors 24 relativ zu dem tragenden Gantryteil T um die Drehachse SA auf, wobei der Drehantrieb 26 mit elektrischer Energie von dem Energiespeicher ET des tragenden Gantryteils T versorgt wird.

Beim Übergang von dem ersten Betriebszustand des Computertomographiegeräts 1 zu dem zweiten Betriebszustand des Computertomographiegeräts 1 wird die Energieübertragungsverbindung durch eine Entkopplung des ersten Verbindungselements C1 und des zweiten Verbindungselements C2 voneinander unterbrochen, indem das erste Verbindungselement C1 und das zweite Verbindungselement C2 mittels der Kopplungsvorrichtung C12 in einer Entkopplungs-Position relativ zueinander angeordnet werden, während der Rotor 24 relativ zu dem tragenden Gantryteil T ruht und sich in der Kopplungs-Winkelposition relativ zu dem tragenden Gantryteil T befindet.

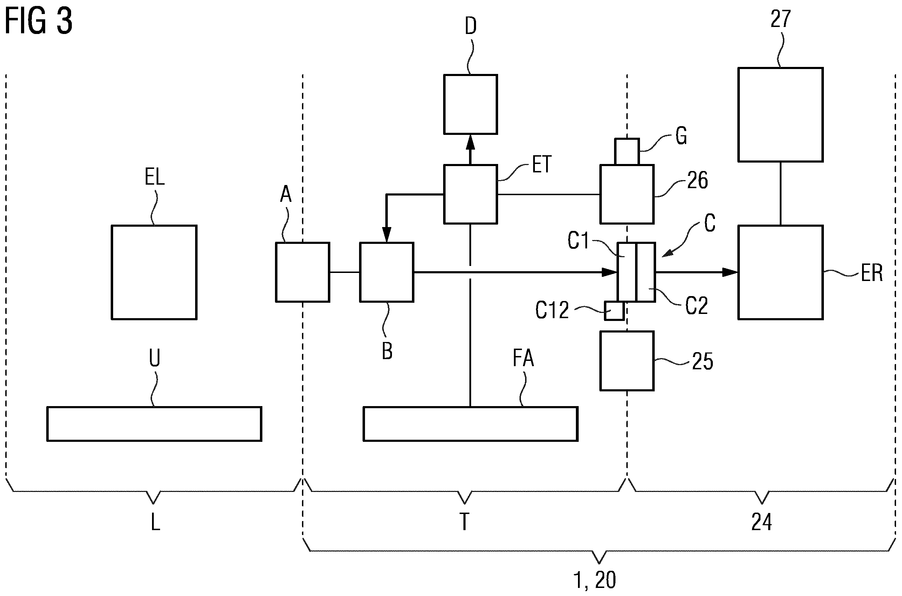

Die Fig. 3 zeigt ein Energieflussdiagramm für einen dritten Betriebszustand des Computertomographiegeräts 1. In dem dritten Betriebszustand des Computertomographiegeräts 1 ruht der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C hergestellt ist. In dem zweiten Betriebszustand des Computertomographiegeräts 1 ist der Anschluss A nicht mit dem elektrischen Energieübertragungsnetz EL verbunden.

Beispielsweise kann auf diese Weise nach einmaligem oder mehrmaligem Erfassen von Projektionsdaten, das in dem zweiten Betriebszustand des Computertomographiegeräts 1 durchgeführt wurde, der Energiespeicher ER des Rotors 24 mit elektrischer Energie aus dem Energiespeicher ET des tragenden Gantryteils T aufgeladen werden.

Beim Übergang von dem zweiten Betriebszustand des Computertomographiegeräts 1 zu dem dritten Betriebszustand des Computertomographiegeräts 1 wird der Rotor 24 in der Kopplungs-Winkelposition relativ zu dem tragenden Gantryteil T mittels der Winkelpositionierungsvorrichtung G derart winkelpositioniert, dass das erste Verbindungselement C1 und das zweite Verbindungselement C2 zueinander für das Herstellen der Energieübertragungsverbindung korrespondierend ausgerichtet sind.

Beim Übergang von dem zweiten Betriebszustand des Computertomographiegeräts 1 zu dem dritten Betriebszustand des Computertomographiegeräts 1 wird die Energieübertragungsverbindung durch eine Kopplung des ersten Verbindungselements C1 und des zweiten Verbindungselements C2 aneinander hergestellt, indem das erste Verbindungselement C1 und das zweite Verbindungselement C2 mittels der Kopplungsvorrichtung C12 in der Kopplungs-Position relativ zueinander angeordnet werden, während der Rotor 24 relativ zu dem tragenden Gantryteil T ruht und sich in der Kopplungs-Winkelposition relativ zu dem tragenden Gantryteil T befindet.

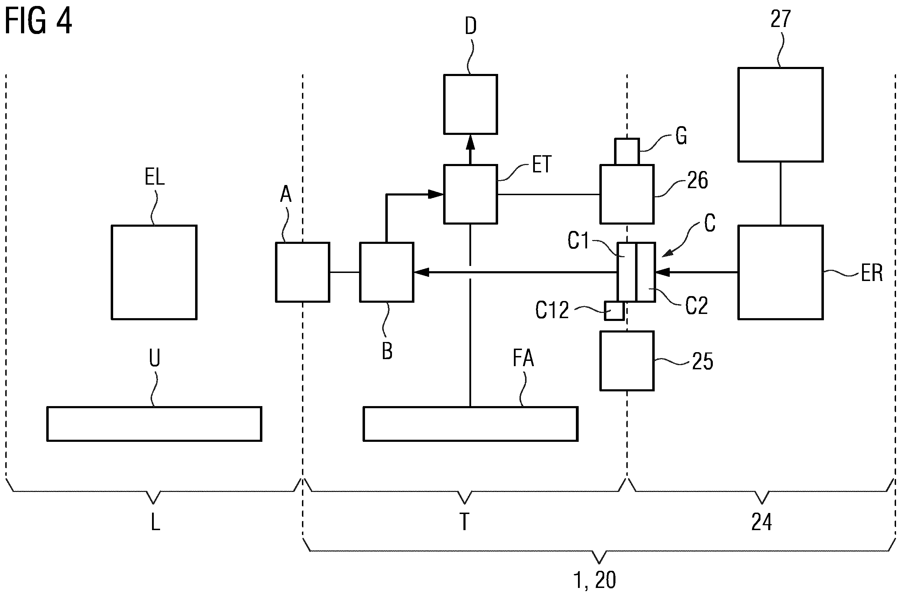

Die Fig. 4 zeigt ein Energieflussdiagramm für einen vierten Betriebszustand des Computertomographiegeräts 1. In dem dritten Betriebszustand des Computertomographiegeräts 1 ruht der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C hergestellt ist. In dem zweiten Betriebszustand des Computertomographiegeräts 1 ist der Anschluss A nicht mit dem elektrischen Energieübertragungsnetz EL verbunden. Das Energieübertragungssystem C ist für eine Energieübertragung von dem Energiespeicher ER des Rotors 24 zu dem tragenden Gantryteil T eingerichtet, wobei während der Energieübertragung von dem Energiespeicher ER des Rotors 24 zu dem tragenden Gantryteil T der Rotor 24 relativ zu dem tragenden Gantryteil T ruht.

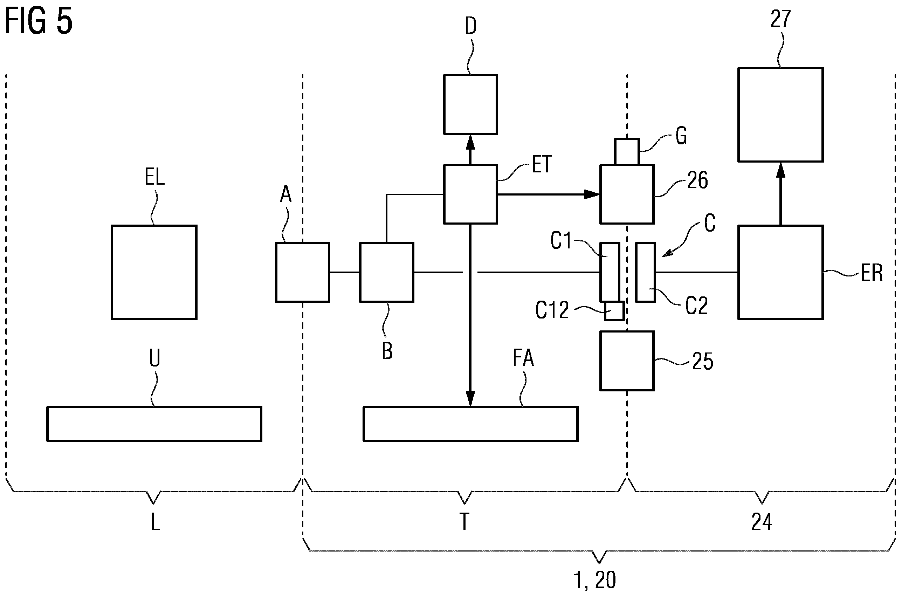

Die Fig. 5 zeigt ein Energieflussdiagramm für einen fünften Betriebszustand des Computertomographiegeräts 1. In dem fünften Betriebszustand des Computertomographiegeräts 1 dreht sich der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C unterbrochen ist. In dem zweiten Betriebszustand des Computertomographiegeräts 1 ist der Anschluss A nicht mit dem elektrischen Energieübertragungsnetz EL verbunden.

Es werden Projektionsdaten mittels des Projektionsdatenakquisitionssystems 27 erfasst, wobei das Projektionsdatenakquisitionssystem 27 mit elektrischer Energie von dem Energiespeicher ER des Rotors 24 versorgt wird. Die Gantry 20 weist den Drehantrieb 26 zum Antreiben der Drehbewegung des Rotors 24 relativ zu dem tragenden Gantryteil T um die Drehachse SA auf, wobei der Drehantrieb 26 mit elektrischer Energie von dem Energiespeicher ET des tragenden Gantryteils T versorgt wird.

Die Gantry 20 weist das Fahrwerk FW auf, welches für eine Scanbewegung der Gantry 20 relativ zu der Grundfläche U eingerichtet ist. Der tragende Gantryteil T weist einen elektrischen Fahrantrieb FA, der mit dem Fahrwerk FW zusammenwirkt, auf. In dem fünften Betriebszustand des Computertomographiegeräts 1 wird während des Erfassens der Projektionsdaten die Scanbewegung der Gantry 20 relativ zu der Grundfläche U basierend auf elektrischer Energie aus dem Energiespeicher ET des tragenden Gantryteils T mittels des Fahrantriebs FA angetrieben.

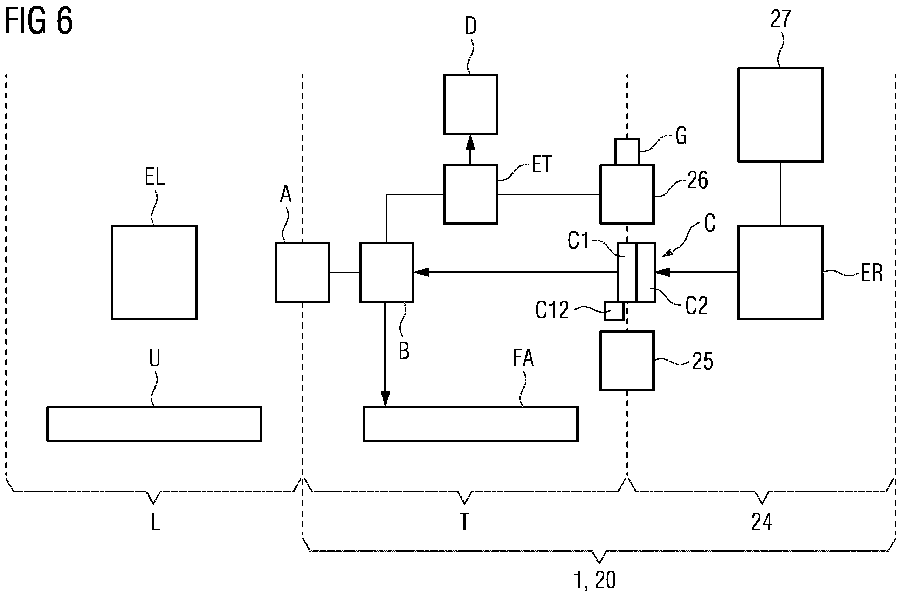

Die Fig. 6 zeigt ein Energieflussdiagramm für einen Betriebszustand eines Computertomographiegeräts 1 gemäß einem weiteren Beispiel. In diesem Betriebszustand des Computertomographiegeräts 1 ruht der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C hergestellt ist und der Anschluss A nicht mit dem elektrischen Energieübertragungsnetz EL verbunden ist.

Die Gantry 20 weist das Fahrwerk FW auf, welches für eine Fahrbewegung der Gantry 20 relativ zu der Grundfläche U eingerichtet ist. Der tragende Gantryteil T weist einen elektrischen Fahrantrieb FA, der mit dem Fahrwerk FW zusammenwirkt, auf. In dem Betriebszustand des Computertomographiegeräts 1 gemäß Fig. 6 wird eine Fahrbewegung der Gantry 20 relativ zu der Grundfläche U mittels des Fahrwerks FW durchgeführt. Die Energieübertragung von dem Energiespeicher ER des Rotors 24 zu dem tragenden Gantryteil T erfolgt während der Fahrbewegung der Gantry 20. Die Energieübertragung von dem Energiespeicher ER des Rotors 24 zu dem tragenden Gantryteil T erfolgt mittels des Energieübertragungssystems C von dem Energiespeicher ER des Rotors 24 zu dem elektrischen Fahrantrieb FA, um die Fahrbewegung der Gantry 20 mittels des Fahrantriebs FA anzutreiben.

Durch die Energieübertragung von dem Energiespeicher ER des Rotors 24 zu dem tragenden Gantryteil T mittels des Energieübertragungssystems C wird somit elektrische Energie für den elektrischen Fahrantrieb FA von dem Energiespeicher ER des Rotors 24 zu dem tragenden Gantryteil T, der mit dem Fahrwerk FW zusammenwirkt, übertragen, wobei die Fahrbewegung der Gantry 20 mittels des elektrischen Fahrantriebs FA basierend auf der elektrischen Energie für den elektrischen Fahrantrieb FA angetrieben wird.

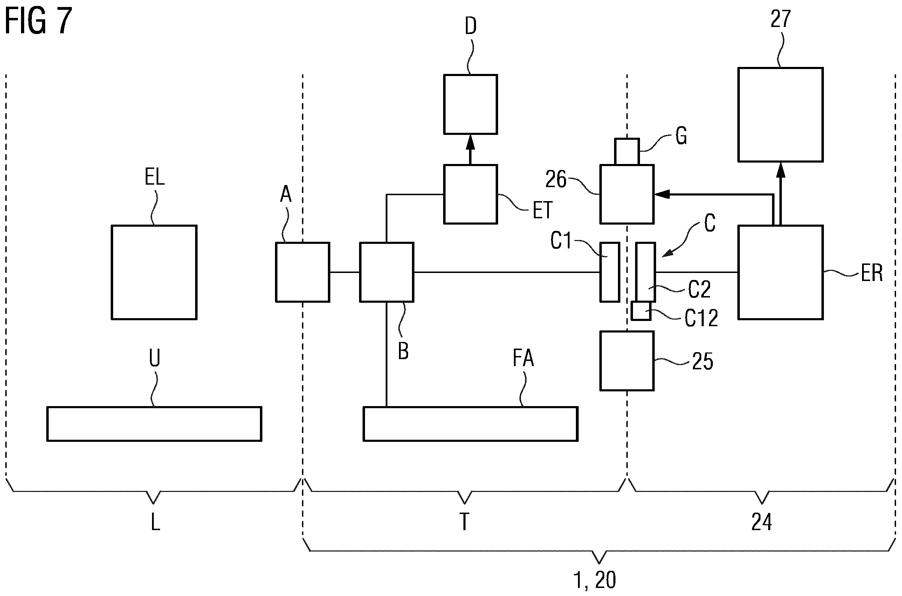

Die Fig. 7 zeigt ein Energieflussdiagramm für einen Betriebszustand eines Computertomographiegeräts 1 gemäß einem weiteren Beispiel. In diesem Betriebszustand des Computertomographiegeräts 1 dreht sich der Rotor 24 relativ zu dem tragenden Gantryteil T, wobei zwischen dem Energiespeicher ER des Rotors 24 und dem tragenden Gantryteil T die Energieübertragungsverbindung des Energieübertragungssystems C unterbrochen ist und der Anschluss A nicht mit dem elektrischen Energieübertragungsnetz EL verbunden ist.

Es werden Projektionsdaten mittels des Projektionsdatenakquisitionssystems 27 erfasst, wobei das Projektionsdatenakquisitionssystem 27 mit elektrischer Energie von dem Energiespeicher ER des Rotors 24 versorgt wird. Die Gantry 20 weist den Drehantrieb 26 zum Antreiben der Drehbewegung des Rotors 24 relativ zu dem tragenden Gantryteil T um die Drehachse SA auf, wobei der Drehantrieb 26 mit elektrischer Energie von dem Energiespeicher ER des Rotors 24 versorgt wird.

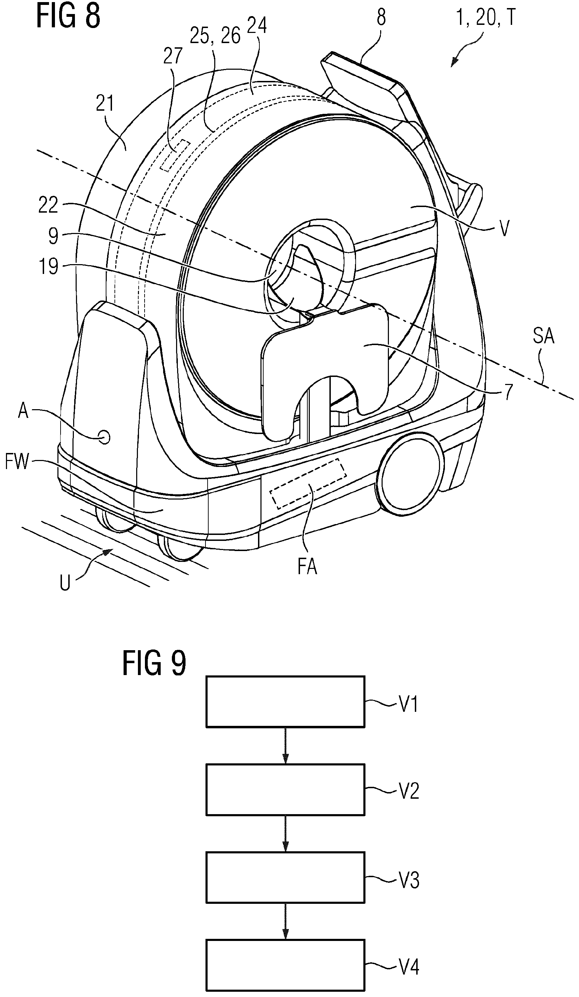

Die Fig. 8 zeigt ein Beispiel für ein Computertomographiegerät 1 mit der Gantry 20. Die Gantry 20 weist einen ersten Gantryteil 21 und einen zweiten Gantryteil 22 auf, wobei der erste Gantryteil 21 den drehbar gelagerten Rotor 24 mit dem Projektionsdatenakquisitionssystem 27 aufweist, wobei der zweite Gantryteil 22 zumindest einen Abschnitt der Öffnung 9 aufweist. Der erste Gantryteil 21 weist die Drehlagerung 25 und eine Tragstruktur 26 auf, wobei der Rotor 24 mittels der Drehlagerung 25 mit der Tragstruktur 26 verbunden und relativ zu der Tragstruktur 26 um die Drehachse SA drehbar gelagert ist, wobei die Drehachse SA eine Systemachse der Gantry 20 ist. Der erste Gantryteil 21 ist relativ zu dem zweiten Gantryteil 22 derart bewegbar gelagert, dass eine Translationsbewegung des ersten Gantryteils 21 relativ zu dem zweiten Gantryteil 22 entlang der Systemachse der Gantry 20 ausgeführt werden kann, während gleichzeitig dazu der zweite Gantryteil 22 relativ zu der Kopfschale 19 und relativ zu dem zumindest einen Abschnitt der Öffnung 9 ruht.

Der tragende Gantryteil T kann beispielsweise den zweiten Gantryteil 22 und die Tragstruktur 26 des ersten Gantryteils 21 aufweisen. Die Translationsbewegung des ersten Gantryteils 21 relativ zu dem zweiten Gantryteil 22 kann beispielsweise mit elektrischer Energie aus dem Energiespeicher ET des tragenden Gantryteils T angetrieben werden, wenn sich der Rotor 24 relativ zu dem tragenden Gantryteil T dreht. Die Translationsbewegung des ersten Gantryteils 21 relativ zu dem zweiten Gantryteil 22 kann beispielsweise mit elektrischer Energie aus dem Energiespeicher ER des Rotors 24 angetrieben werden, wenn der Rotor 24 relativ zu dem tragenden Gantryteil T ruht.

Das in der Fig. 8 gezeigte Computertomographiegerät 1 ist ein mobiles Kopf-Computertomographiegerät. Die Gantry 20 weist die Kopfschale 19 und die Körperstützvorrichtung 7 auf. Auf der Kopfschale 19 kann ein Kopf eines Menschen ruhen, während sich der Kopf des Menschen für eine Kopfbildgebung in der Öffnung 9 befindet. Die Körperstützvorrichtung 7 kann zur Stützung eines Schulterbereichs des Menschen verwendet werden, während sich der Kopf des Menschen für die Kopfbildgebung in der Öffnung 9 befindet und auf der Kopfschale 19 ruht. Die Gantry 20 weist ferner eine Verkleidung V zum Abgrenzen eines Innenbereichs der Gantry 20 von einer Umgebung L der Gantry 20 auf. Das Computertomographiegerät 1 weist ferner ein Bediensystem mit einem berührungsempfindlichen Bildschirm 8, der an der Gantry 20 angeordnet ist, auf.

Die Fig. 9 zeigt ein Ablaufdiagramm eines Verfahrens zur Energieübertragung in einem Computertomographiegerät 1, wobei das Computertomographiegerät 1 eine Gantry 20 mit einem tragenden Gantryteil T, einem Rotor 24 und einem Energieübertragungssystem C aufweist, wobei der Rotor 24 das Projektionsdatenakquisitionssystem 27 und einen Energiespeicher ER aufweist und relativ zu dem tragenden Gantryteil T drehbar gelagert ist, das Verfahren umfassend:
- ein Herbeiführen V1 eines Ruhezustands des Rotors 24 relativ zu dem tragenden Gantryteil T derart, dass der Rotor 24 relativ zu dem tragenden Gantryteil T ruht,
- ein Durchführen V2 einer Energieübertragung von dem tragenden Gantryteil T zu dem Energiespeicher ER des Rotors 24 mittels des Energieübertragungssystems C, während der Rotor 24 relativ zu dem tragenden Gantryteil T ruht,
- ein Drehen V3 des Rotors 24 relativ zu dem tragenden Gantryteil T,
- ein Versorgen V4 des Projektionsdatenakquisitionssystems 27 mit elektrischer Energie aus dem Energiespeicher ER des Rotors 24 während des Drehens V3 des Rotors 24 relativ zu dem tragenden Gantryteil T.

## Patentansprüche

1. Computertomographiegerät (1), aufweisend eine Gantry (20) mit einem tragenden Gantryteil (T) und einem Rotor (24),
- wobei der Rotor (24) ein Projektionsdatenakquisitionssystem (27) und einen Energiespeicher (ER) zur Energieversorgung des Projektionsdatenakquisitionssystems (27) aufweist und relativ zu dem tragenden Gantryteil (T) drehbar gelagert ist,
- wobei die Gantry (20) ein Energieübertragungssystem (C) aufweist, welches für eine Energieübertragung von dem tragenden Gantryteil (T) zu dem Energiespeicher (ER) des Rotors (24) eingerichtet ist, wobei während der Energieübertragung von dem tragenden Gantryteil (T) zu dem Energiespeicher (ER) des Rotors (24) der Rotor (24) relativ zu dem tragenden Gantryteil (T) ruht,
- **dadurch gekennzeichnet, dass** das Energieübertragungssystem (C) ferner für eine Energieübertragung von dem Energiespeicher (ER) des Rotors (24) zu dem tragenden Gantryteil (T) eingerichtet ist, wobei während der Energieübertragung von dem Energiespeicher (ER) des Rotors (24) zu dem tragenden Gantryteil (T) der Rotor (24) relativ zu dem tragenden Gantryteil (T) ruht.

2. Computertomographiegerät (1) nach Anspruch 1,
- wobei die Gantry (20) ein Fahrwerk (FW) aufweist, welches für eine Fahrbewegung der Gantry (20) relativ zu einer Grundfläche (U) eingerichtet ist,
- wobei die Energieübertragung von dem Energiespeicher (ER) des Rotors (24) zu dem tragenden Gantryteil (T) während der Fahrbewegung der Gantry (20) erfolgt.

3. Computertomographiegerät (1) nach Anspruch 2,
- wobei der tragende Gantryteil (T) einen elektrischen Fahrantrieb (FA), der mit dem Fahrwerk (FW) zusammenwirkt, aufweist,
- wobei die Energieübertragung von dem Energiespeicher (ER) des Rotors (24) zu dem tragenden Gantryteil (T) mittels des Energieübertragungssystems (C) von dem Energiespeicher (ER) des Rotors (24) zu dem elektrischen Fahrantrieb (FA) erfolgt, um die Fahrbewegung der Gantry (20) mittels des Fahrantriebs (FA) anzutreiben.

4. Computertomographiegerät (1) nach einem der Ansprüche 1 bis 3,
- wobei der tragende Gantryteil (T) einen Energiespeicher (ET) aufweist,
- wobei die Energieübertragung von dem tragenden Gantryteil (T) zu dem Energiespeicher (ER) des Rotors (24) mittels des Energieübertragungssystems (C) von dem Energiespeicher (ET) des tragenden Gantryteils (T) zu dem Energiespeicher (ER) des Rotors (24) erfolgt und/oder wobei die Energieübertragung von dem Energiespeicher (ER) des Rotors (24) zu dem tragenden Gantryteil (T) mittels des Energieübertragungssystems (C) von dem Energiespeicher (ER) des Rotors (24) zu dem Energiespeicher (ET) des tragenden Gantryteils (T) erfolgt.

5. Computertomographiegerät (1) nach einem der Ansprüche 1 bis 4,
- wobei der tragende Gantryteil (T) ein erstes Verbindungselement (C1) des Energieübertragungssystems (C) aufweist,
- wobei der Rotor (24) ein zweites Verbindungselement (C2) des Energieübertragungssystems (C) aufweist,
- wobei das erste Verbindungselement (C1) und das zweite Verbindungselement (C2) zueinander für ein Herstellen einer Energieübertragungsverbindung korrespondierend ausgebildet sind,
- wobei das erste Verbindungselement (C1) und das zweite Verbindungselement (C2) zueinander für das Herstellen der Energieübertragungsverbindung korrespondierend ausgerichtet sind, wenn sich der Rotor (24) in einer Kopplungs-Winkelposition relativ zu dem tragenden Gantryteil (T) befindet.

6. Computertomographiegerät (1) nach Anspruch 5,
- wobei die Energieübertragungsverbindung eine elektrische Steckverbindung des ersten Verbindungselements (C1) und des zweiten Verbindungselements (C2) miteinander ist.

7. Computertomographiegerät (1) nach Anspruch 5,
- wobei die Energieübertragungsverbindung zur kontaktlosen elektrischen Energieübertragung zwischen dem ersten Verbindungselement (C1) und dem zweiten Verbindungselement (C2) eingerichtet ist.

8. Computertomographiegerät (1) nach einem der Ansprüche 5 bis 7,
- wobei die Gantry (20) eine Kopplungsvorrichtung (C12) aufweist, welche zum Anordnen des ersten Verbindungselements (C1) und des zweiten Verbindungselements (C2) in einer Kopplungs-Position relativ zueinander, während der Rotor (24) relativ zu dem tragenden Gantryteil (T) ruht, und zum Anordnen des ersten Verbindungselements (C1) und des zweiten Verbindungselements (C2) in einer Entkopplungs-Position relativ zueinander, während der Rotor (24) relativ zu dem tragenden Gantryteil (T) ruht, eingerichtet ist,
- wobei die Energieübertragungsverbindung durch eine Kopplung des ersten Verbindungselements (C1) und des zweiten Verbindungselements (C2) aneinander hergestellt ist, wenn sich der Rotor (24) in der Kopplungs-Winkelposition relativ zu dem tragenden Gantryteil (T) befindet und wenn das erste Verbindungselement (C1) und das zweite Verbindungselement (C2) in der Kopplungs-Position relativ zueinander angeordnet sind,
- wobei die Energieübertragungsverbindung durch eine Entkopplung des ersten Verbindungselements (C1) und des zweiten Verbindungselements (C2) voneinander unterbrochen ist, wenn das erste Verbindungselement (C1) und das zweite Verbindungselement (C2) in der Entkopplungs-Position relativ zueinander angeordnet sind.

9. Verfahren zur Energieübertragung in einem Computertomographiegerät (1), wobei das Computertomographiegerät (1) eine Gantry (20) mit einem tragenden Gantryteil (T), einem Rotor (24) und einem Energieübertragungssystem (C) aufweist, wobei der Rotor (24) das Projektionsdatenakquisitionssystem (27) und einen Energiespeicher (ER) aufweist und relativ zu dem tragenden Gantryteil (T) drehbar gelagert ist, das Verfahren umfassend:
- ein Herbeiführen (V1) eines Ruhezustands des Rotors (24) relativ zu dem tragenden Gantryteil (T) derart, dass der Rotor (24) relativ zu dem tragenden Gantryteil (T) ruht,
- ein Durchführen (V2) einer Energieübertragung von dem tragenden Gantryteil (T) zu dem Energiespeicher (ER) des Rotors (24) mittels des Energieübertragungssystems (C), während der Rotor (24) relativ zu dem tragenden Gantryteil (T) ruht,
- ein Drehen (V3) des Rotors (24) relativ zu dem tragenden Gantryteil (T),
- ein Versorgen (V4) des Projektionsdatenakquisitionssystems (27) mit elektrischer Energie aus dem Energiespeicher (ER) des Rotors (24) während des Drehens (V3) des Rotors (24) relativ zu dem tragenden Gantryteil (T,
- **dadurch gekennzeichnet, dass** eine Energieübertragung von dem Energiespeicher (ER) des Rotors (24) zu dem tragenden Gantryteil (T) mittels des Energieübertragungssystems (C) durchgeführt wird, während der Rotor (24) relativ zu dem tragenden Gantryteil (T) ruht.

10. Verfahren nach Anspruch 9,
- wobei eine Fahrbewegung der Gantry (20) relativ zu einer Grundfläche (U) mittels eines Fahrwerks (FW) durchgeführt wird,
- wobei die Energieübertragung von dem Energiespeicher (ER) des Rotors (24) zu dem tragenden Gantryteil (T) während der Fahrbewegung der Gantry (20) durchgeführt wird.

11. Verfahren nach Anspruch 10,
- wobei durch die Energieübertragung von dem Energiespeicher (ER) des Rotors (24) zu dem tragenden Gantryteil (T) mittels des Energieübertragungssystems (C) elektrische Energie für einen elektrischen Fahrantrieb (FA), der mit dem Fahrwerk (FW) zusammenwirkt, von dem Energiespeicher (ER) des Rotors (24) zu dem tragenden Gantryteil (T) übertragen wird,
- wobei die Fahrbewegung der Gantry (20) mittels des elektrischen Fahrantriebs (FA) basierend auf der elektrischen Energie für den elektrischen Fahrantrieb (FA) angetrieben wird.

12. Verfahren nach einem der Ansprüche 9 bis 11,
- wobei der tragende Gantryteil (T) ein erstes Verbindungselement (C1) des Energieübertragungssystems (C) aufweist, wobei der Rotor (24) ein zweites Verbindungselement (C2) des Energieübertragungssystems (C) aufweist, wobei das erste Verbindungselement (C1) und das zweite Verbindungselement (C2) zueinander für ein Herstellen einer Energieübertragungsverbindung korrespondierend ausgebildet sind,
- wobei der Rotor (24) in einer Kopplungs-Winkelposition relativ zu dem tragenden Gantryteil (T) derart winkelpositioniert wird, dass das erste Verbindungselement (C1) und das zweite Verbindungselement (C2) zueinander für das Herstellen der Energieübertragungsverbindung korrespondierend ausgerichtet sind.

13. Verfahren nach Anspruch 12,
- wobei die Energieübertragungsverbindung durch eine Kopplung des ersten Verbindungselements (C1) und des zweiten Verbindungselements (C2) aneinander hergestellt wird, indem das erste Verbindungselement (C1) und das zweite Verbindungselement (C2) in einer Kopplungs-Position relativ zueinander angeordnet werden, während der Rotor (24) relativ zu dem tragenden Gantryteil (T) ruht und sich in der Kopplungs-Winkelposition relativ zu dem tragenden Gantryteil (T) befindet,
- wobei die Energieübertragungsverbindung durch eine Entkopplung des ersten Verbindungselements (C1) und des zweiten Verbindungselements (C2) voneinander unterbrochen wird, indem das erste Verbindungselement (C1) und das zweite Verbindungselement (C2) in einer Entkopplungs-Position relativ zueinander angeordnet werden, während der Rotor (24) relativ zu dem tragenden Gantryteil (T) ruht und sich in der Kopplungs-Winkelposition relativ zu dem tragenden Gantryteil (T) befindet.

## Claims

1. Computed tomography device (1), having a gantry (20) with a supporting gantry part (T) and a rotor (24),
- wherein the rotor (24) has a projection data acquisition system (27) and an energy store (ER) for supplying energy to the projection data acquisition system (27) and is rotatably mounted relative to the supporting gantry part (T),
- wherein the gantry (20) has an energy transmission system (C) which is configured for energy transmission from the supporting gantry part (T) to the energy store (ER) of the rotor (24), wherein during the energy transmission from the supporting gantry part (T) to the energy store (ER) of the rotor (24), the rotor (24) is at rest relative to the supporting gantry part (T),
- **characterised in that** the energy transmission system (C) is further configured for energy transmission from the energy store (ER) of the rotor (24) to the supporting gantry part (T), wherein during the energy transmission from the energy store (ER) of the rotor (24) to the supporting gantry part (T), the rotor (24) is at rest relative to the supporting gantry part (T).

2. Computed tomography device (1) according to claim 1,
- wherein the gantry (20) has a chassis (FW) which is configured for a travel movement of the gantry (20) relative to a base area (U),
- wherein the energy transmission from the energy store (ER) of the rotor (24) to the supporting gantry part (T) takes place during the travel movement of the gantry (20).

3. Computed tomography device (1) according to claim 2,
- wherein the supporting gantry part (T) has an electric travel drive (FA) which interacts with the chassis (FW),
- wherein the energy transmission from the energy store (ER) of the rotor (24) to the supporting gantry part (T) takes place by means of the energy transmission system (C) from the energy store (ER) of the rotor (24) to the electric travel drive (FA) in order to drive the travel movement of the gantry (20) by means of the travel drive (FA).

4. Computed tomography device (1) according to one of claims 1 to 3,
- wherein the supporting gantry part (T) has an energy store (ET),
- wherein the energy transmission from the supporting gantry part (T) to the energy store (ER) of the rotor (24) takes place by means of the energy transmission system (C) from the energy store (ET) of the supporting gantry part (T) to the energy store (ER) of the rotor (24) and/or wherein the energy transmission from the energy store (ER) of the rotor (24) to the supporting gantry part (T) takes place by means of the energy transmission system (C) from the energy store (ER) of the rotor (24) to the energy store (ET) of the supporting gantry part (T).

5. Computed tomography device (1) according to one of claims 1 to 4,
- wherein the supporting gantry part (T) has a first connecting element (C1) of the energy transmission system (C),
- wherein the rotor (24) has a second connecting element (C2) of the energy transmission system (C),
- wherein the first connecting element (C1) and the second connecting element (C2) are aligned correspondingly to one another for establishing an energy transmission connection,
- wherein the first connecting element (C1) and the second connecting element (C2) are aligned correspondingly to one another for establishing the energy transmission connection when the rotor (24) is in a coupling angular position relative to the supporting gantry part (T).

6. Computed tomography device (1) according to claim 5,
- wherein the energy transmission connection is an electrical plug connection of the first connecting element (C1) and of the second connecting element (C2) to one another.

7. Computed tomography device (1) according to claim 5,
- wherein the energy transmission connection is configured for contactless electrical energy transmission between the first connecting element (C1) and the second connecting element (C2).

8. Computed tomography device (1) according to one of claims 5 to 7,
- wherein the gantry (20) has a coupling apparatus (C12) which is configured to arrange the first connecting element (C1) and the second connecting element (C2) in a coupling position relative to one another, while the rotor (24) is at rest relative to the supporting gantry part (T), and to arrange the first connecting element (C1) and the second connecting element (C2) in a decoupling position relative to one another while the rotor (24) is at rest relative to the supporting gantry part (T),
- wherein the energy transmission connection is established by coupling the first connecting element (C1) and the second connecting element (C2) to one another when the rotor (24) is in the coupling angular position relative to the supporting gantry part (T) and when the first connecting element (C1) and the second connecting element (C2) are arranged in the coupling position relative to one another,
- wherein the energy transmission connection is interrupted by decoupling the first connecting element (C1) and the second connecting element (C2) from one another when the first connecting element (C1) and the second connecting element (C2) are arranged in the decoupling position relative to one another.

9. Method for energy transmission in a computed tomography device (1), wherein the computed tomography device (1) has a gantry (20) with a supporting gantry part (T), a rotor (24) and an energy transmission system (C), wherein the rotor (24) has the projection data acquisition system (27) and an energy store (ER) and is rotatably mounted relative to the supporting gantry part (T), the method comprising:
- causing (V1) an idle state of the rotor (24) relative to the supporting gantry part (T) in such a way that the rotor (24) is at rest relative to the supporting gantry part (T),
- carrying out (V2) energy transmission from the supporting gantry part (T) to the energy store (ER) of the rotor (24) by means of the energy transmission system (C) while the rotor (24) is at rest relative to the supporting gantry part (T),
- rotating (V3) the rotor (24) relative to the supporting gantry part (T),
- supplying (V4) the projection data acquisition system (27) with electrical energy from the energy store (ER) of the rotor (24) during rotation (V3) of the rotor (24) relative to the supporting gantry part (T),
- **characterised in that** energy transmission from the energy store (ER) of the rotor (24) to the supporting gantry part (T) is performed by means of the energy transmission system (C) while the rotor (24) is at rest relative to the supporting gantry part (T).

10. Method according to claim 9,
- wherein a travel movement of the gantry (20) relative to a base area (U) is carried out by means of a chassis (FW),
- wherein the energy transmission from the energy store (ER) of the rotor (24) to the supporting gantry part (T) is carried out during the travel movement of the gantry (20).

11. Method according to claim 10,
- wherein, as a result of the energy transmission from the energy store (ER) of the rotor (24) to the supporting gantry part (T) by means of the energy transmission system (C), electrical energy for an electric travel drive (FA) which interacts with the chassis (FW) is transmitted from the energy store (ER) of the rotor (24) to the supporting gantry part (T),
- wherein the travel movement of the gantry (20) is driven by means of the electric travel drive (FA) based on the electrical energy for the electric travel drive (FA).

12. Method according to one of claims 9 to 11,
- wherein the supporting gantry part (T) has a first connecting element (C1) of the energy transmission system (C), wherein the rotor (24) has a second connecting element (C2) of the energy transmission system (C), wherein the first connecting element (C1) and the second connecting element (C2) are aligned correspondingly to one another for establishing an energy transmission connection,
- wherein the rotor (24) is angularly positioned in a coupling angular position relative to the supporting gantry part (T) in such a way that the first connecting element (C1) and the second connecting element (C2) are aligned correspondingly to one another for establishing the energy transmission connection.

13. Method according to claim 12,
- wherein the energy transmission connection is established by coupling the first connecting element (C1) and the second connecting element (C2) to one another by arranging the first connecting element (C1) and the second connecting element (C2) in a coupling position relative to one another while the rotor (24) is at rest relative to the supporting gantry part (T) and is in the coupling angular position relative to the supporting gantry part (T),
- wherein the energy transmission connection is interrupted by decoupling the first connecting element (C1) and the second connecting element (C2) from one another by arranging the first connecting element (C1) and the second connecting element (C2) in a decoupling position relative to one another while the rotor (24) is at rest relative to the supporting gantry part (T) and is in the coupling angular position relative to the supporting gantry part (T).

## Revendications

1. Appareil (1) de tomodensitométrie assisté par ordinateur, comportant un portique (20) ayant une partie (T) de portique portante et un rotor (24),
- dans lequel le rotor (24) a un système (27) d'acquisition de données de projection et un accumulateur (ER) d'énergie électrique pour l'alimentation en énergie électrique du système (27) d'acquisition de données de projection et est monté tournant par rapport à la partie (T) de portique portante,
- dans lequel le portique (20) a un système (C) de transfert d'énergie électrique, qui est agencé pour un transfert d'énergie de la partie (T) de portique portante à l'accumulateur (ER) d'énergie du rotor (24), dans lequel pendant le transfert d'énergie de la partie (T) de portique portante à l'accumulateur (ER) d'énergie du rotor (24), le rotor (24) est au repos relativement à la partie (T) de portique portante,
- **caractérisé en ce que** le système (C) de transfert d'énergie est agencé en outre pour un transfert d'énergie de l'accumulateur (ER) d'énergie du rotor (24) à la partie (T) de portique portante, dans lequel pendant le transfert d'énergie de l'accumulateur (ER) d'énergie du rotor (24) à la partie (T) de portique portante, le rotor (24) est au repos relativement à la partie (T) de portique portante.

2. Appareil (1) de tomodensitométrie assisté par ordinateur suivant la revendication 1,
- dans lequel le portique (20) a un châssis (FW), qui est agencé pour un mouvement de déplacement du portique (20) relativement à une surface (U) du sol,
- dans lequel le transfert d'énergie de l'accumulateur (ER) d'énergie du rotor (24) à la partie (T) de portique portante s'effectue pendant le mouvement de déplacement du portique (20).

3. Appareil (1) de tomodensitométrie assisté par ordinateur suivant la revendication 2,
- dans lequel la partie (T) de portique portante a un entraînement (FA) électrique de déplacement en translation, qui coopère avec le châssis (FW),
- dans lequel le transfert d'énergie de l'accumulateur (ER) du rotor (24) à la partie (T) du portique tournante s'effectue au moyen du système (C) de transfert d'énergie de l'accumulateur (ER) d'énergie du rotor (24) à l'entraînement (FA) électrique de mouvement en translation, afin de provoquer le mouvement de translation du portique (20) au moyen de l'entraînement (FA) de mouvement en translation.

4. Appareil (1) de tomodensitométrie assisté par ordinateur suivant l'une des revendications 1 à 3,
- dans lequel la partie (T) de portique portante a un accumulateur (ET) d'énergie,
- dans lequel le transfert d'énergie de la partie (T) de portique portante à l'accumulateur (ER) d'énergie du rotor (24) s'effectue au moyen du système (C) de transfert d'énergie de l'accumulateur (ET) d'énergie de la partie (T) de portique portante à l'accumulateur (ER) d'énergie du rotor (24), et/ou dans lequel le transfert d'énergie de l'accumulateur (ER) d'énergie du rotor (24) à la partie (T) de portique portante s'effectue au moyen du système (C) de transfert d'énergie de l'accumulateur (ER) d'énergie du rotor (24) à l'accumulateur (ET) d'énergie de la partie (T) de portique portante.

5. Appareil (1) de tomodensitométrie assisté par ordinateur suivant l'une des revendications 1 à 4,
- dans lequel la partie (T) de portique portante a un premier élément (C1) de connexion du système (C) de transfert d'énergie,
- dans lequel le rotor (24) a un deuxième élément (C2) de connexion du système (C) de transfert d'énergie,
- dans lequel le premier élément (C1) de connexion et le deuxième élément (C2) de connexion sont constitués de manière à se correspondre l'un à l'autre pour une production d'une connexion de transfert d'énergie,
- dans lequel le premier élément (C1) de connexion et le deuxième élément (C2) de connexion sont agencés pour se correspondre l'un à l'autre pour la production de la connexion de transfert d'énergie, si le rotor (24) se trouve dans une position angulaire d'accouplement relativement à la partie (T) de portique portante.

6. Appareil (1) de tomodensitométrie assisté par ordinateur suivant la revendication 5,
- dans lequel la connexion de transfert d'énergie est une connexion par enfichage entre eux du premier élément (C1) de connexion et du deuxième élément (C2) de connexion.

7. Appareil (1) de tomodensitométrie assisté par ordinateur suivant la revendication 5,
- dans lequel la connexion de transfert d'énergie est agencée pour le transfert d'énergie électrique sans contact entre le premier élément (C1) et le deuxième élément (C2) de connexion.

8. Appareil (1) de tomodensitométrie assisté par ordinateur suivant l'une des revendications 5 à 7,
- dans lequel le portail (20) a un dispositif (C12) de connexion, qui est agencé pour le montage du premier élément (C1) de connexion et du deuxième élément (C2) de connexion dans une position d'accouplement l'un par rapport à l'autre, pendant que le rotor (24) est au repos relativement à la partie (T) de portique portante, et pour le montage du premier élément (C1) de connexion et du deuxième élément (C2) de connexion dans une position de désaccouplement l'un par rapport à l'autre, pendant que le rotor (24) est au repos relativement à la partie (T) de portique portante,
- dans lequel la connexion de transfert d'énergie est produite par un accouplement du premier élément (C1) de connexion et du deuxième élément (C2) de connexion l'un à l'autre, lorsque le rotor (24) se trouve dans la position angulaire d'accouplement relativement à la partie (T) de portique portante et lorsque le premier élément (C1) de connexion et le deuxième élément (C2) de connexion sont disposés l'un par rapport à l'autre dans la position d'accouplement,
- dans lequel la connexion de transfert d'énergie est interrompue par un désaccouplement du premier élément (C1) de connexion et du deuxième élément (C2) de connexion l'un de l'autre, lorsque le premier élément (C1) de connexion et le deuxième élément (C2) de connexion sont montés l'un relativement à l'autre dans la position de désaccouplement.

9. Procédé de transfert d'énergie dans un appareil (1) de tomodensitométrie assisté par ordinateur, dans lequel l'appareil (1) de tomodensitométrie assisté par ordinateur a un portique (20) ayant une partie (T) de portique portante, un rotor (24) et un système (C) de transfert d'énergie électrique, dans lequel le rotor (24) a le système (27) d'acquisition de données de projection et un accumulateur (ER) d'énergie et est monté tournant relativement à la partie (T) de portique portante, le procédé comprenant :
- faire en sorte (V1) que le rotor (24) prenne un état de repos relativement à la partie (T) de portique portante, de manière à ce que le rotor (24) soit au repos relativement à la partie (T) de portique portante,
- effectuer (V2) un transfert d'énergie de la partie (T) de portique portante à l'accumulateur (ER) d'énergie du rotor (24) au moyen du système (C) de transfert d'énergie, pendant que le rotor (24) est au repos relativement à la partie (T) de portique portante,
- faire tourner (V3) le rotor (24) relativement à la partie (T) de portique portante,
- alimenter (V4) le système (27) d'acquisition de données de projection en énergie électrique à partir de l'accumulateur (ER) d'énergie du rotor (24) pendant la rotation (V3) du rotor (24) relativement à la partie (T) de portique portante,
- **caractérisé en ce que** l'on effectue au moyen du système (C) de transfert d'énergie un transfert d'énergie de l'accumulateur (ER) d'énergie du rotor (24) à la partie (T) de portique portante, pendant que le rotor (24) est au repos relativement à la partie (T) de portique portante.

10. Procédé suivant la revendication 9,
- dans lequel on effectue un mouvement de translation du portique (20) par rapport à une surface (U) du sol au moyen d'un dispositif (FW) de mise en translation,
- dans lequel on effectue le transfert d'énergie de l'accumulateur (ER) d'énergie du rotor (24) à la partie (T) de portique portante pendant le mouvement de translation du portique (20).

11. Procédé suivant la revendication 10,
- dans lequel, par le transfert d'énergie de l'accumulateur (ER) d'énergie du rotor (24) la partie (T) de portique portante, on transfère, au moyen du système (C) de transfert d'énergie, de l'énergie électrique pour un entraînement (FA) électrique en translation, qui coopère avec le dispositif (FW) de déplacement en translation, de l'accumulateur (ER) d'énergie du rotor (24) à la partie (T) de portique portante,
- dans lequel on entraîne le mouvement en translation du portique (20) au moyen de l'entraînement (FA) électrique en translation sur la base de l'énergie électrique pour l'entraînement (FA) électrique en translation.

12. Procédé suivant l'une des revendications 9 à 11,
- dans lequel la partie (T) de portique portante a un premier élément (C1) de connexion du système (C) de transfert d'énergie, dans lequel le rotor (24) a un deuxième élément (C2) de connexion du système (C) de transfert d'énergie, dans lequel le premier élément (C1) de connexion et le deuxième élément (C2) de connexion sont constitués de manière à se correspondre l'un à l'autre pour une production d'une connexion de transfert d'énergie,
- dans lequel on met en position angulaire le rotor (24) dans une position angulaire d'accouplement relativement à la partie (T) de portique portante, de manière à ce que le premier élément (C1) de connexion et le deuxième élément (C2) de connexion soient dirigés de manière correspondante l'un par rapport à l'autre pour la production de la connexion de transfert d'énergie.

13. Procédé suivant la revendication 12,
- dans lequel on produit la connexion de transfert d'énergie par un accouplement du premier élément (C1) de connexion et du deuxième élément (C2) de connexion l'un à l'autre, par le fait que le premier élément (C1) de connexion et le deuxième élément (C2) de connexion sont montés relativement l'un à l'autre dans une position d'accouplement, pendant que le rotor (24) est au repos relativement à la partie (T) de portique portante et se trouve dans la position angulaire d'accouplement relativement à la partie (T) de portique portante,
- dans lequel on interrompt la connexion de transfert d'énergie par un désaccouplement du premier élément (C1) de connexion et du deuxième élément (C2) de connexion l'un de l'autre, par le fait que l'on met le premier élément (C1) de connexion et le deuxième élément (C2) de connexion dans une position de désaccouplement relativement l'un à l'autre, pendant que le rotor (24) est au repos relativement à la partie (T) de portique portante et se trouve dans la position angulaire d'accouplement relativement à la partie (T) de portique portante.
